# EUROPEAN PATENT SPECIFICATION

(11) **EP 3 880 037 B1**
(45) Date of publication and mention of the grant of the patent: **01.05.2024**
(21) Application number: 19884207.2
(22) Date of filing: 08.10.2019
(51) Int. Cl.: A47G 9/02, B32B 3/06, B32B 3/26, B32B 7/04, B32B 7/08, A61M 21/02, D02G 3/00

(54) **LAYERED YARN AND WEIGHTED BLANKET FOR DEEP PRESSURE THERAPY**
GESCHICHTETES GARN UND GEWICHTETE DECKE FÜR TIEFDRUCKTHERAPIE
FIL EN COUCHES ET COUVERTURE LESTÉE POUR UNE THÉRAPIE PAR PRESSION PROFONDE

(30) Priority: 16.11.2018 US 201816193792
(43) Date of publication of application: 22.09.2021
(73) Proprietor: Somnos International Holding Ltd., Dubai P.O. Box 506850 (AE)
(72) Inventor: HAMM, Kathrin, New York, NY 10007 (US)
(74) Representative: Wright, Howard Hugh Burnby
(86) International application number: PCT/US2019/055194
(87) International publication number: WO 2020/101823

(56) References cited:
- US-A- 4 958 663
- US-A1- 2001 053 646
- US-A1- 2006 135 021
- US-A1- 2011 047 698
- US-A1- 2017 295 887
- US-A1- 2017 295 887
- US-A1- 2018 305 847
- US-B2- 6 440 556
- US-B2- 9 956 129
- "GIGANTO-BLANKET FAQ", NOCTURNAL KNITS, 17 October 2011 (2011-10-17), pages 1-9, XP055815784, Retrieved from the Internet: URL:https://nocturnalknits.com/giganto-bla nket-faq [retrieved on 2019-11-22]

## Description

### CROSS REFERENCE TO PRIOR APPLICATIONS

This application claims the benefit of U.S. Patent Application No. 16/193,792 filed on November 16, 2018.

### BACKGROUND

### 1. Field of the Disclosure

The disclosure is directed generally to a layered yarn and weighted blanket. More specifically, the disclosure is directed generally to a layered yarn and weighted blanket for deep pressure therapy. In particular aspects, the disclosure is directed generally to a weighted material that is configured to be used as a weighted blanket to provide a person with deep pressure therapy. It is widely accepted in the medical community that deep pressure therapy may bring relief to those suffering from various disorders, such as insomnia, anxiety, sensory disorders, and the like. US 2018/0305847 A1 discloses a yarn formed by intertwining a plurality of cotton bars with each other.

### 2. Related Art

Some techniques of deep pressure therapy involve placing across a body, a blanket that has weights in it to apply pressure to the body, stimulating a feeling of safety that is also experienced as being hugged or swaddled. In order to be effective, the blanket needs to weigh 10-20% of the person's body weight, leading to the blanket weighing 2,27 to 20,42 kg (5 to 45 pounds).

Conventional deep pressure therapy blankets are typically weighted blankets that are made of a plurality of fabric layers with added weighted materials disposed between the layers. The added weighted materials typically include, for example, plastic pellets or balls, glass beads, sand, gravel, linked chain objects, and the like. The added weighted materials are usually placed in units inside the blanket. For example, the added weighted materials are usually placed in quadratic patches that are sewn or stitched to hold the weights. In these conventional approaches, the added materials are required to provide weight because the conventional fabric and multiple layers of fabric are not heavy enough to effectively provide deep pressure therapy when placed on a person.

The need for the added weights, however, carries with it several disadvantages. For example, the added weights limit breathability of the blanket. The added weights and the multiple layers of fabric reduce natural airflow through the blanket. This makes it particularly difficult to regulate a body temperature of an individual when the blanket is placed on them.

As another example, the added weights are prone to moving and/or shifting within the blanket. This substantially impairs equal, even, and/or continuous weight distribution, which is needed to effectuate deep pressure therapy. Also, the added weights typically cause the blanket to be overly thick and less bendable, preventing the blanket from naturally taking the body shape of a person lying under it and thereby diminishing the surface contact area of the deep pressure therapy.

Accordingly, what is needed is a weighted material that can be used as a blanket to effectively produce deep pressure therapy to an individual, without the need for additional weight materials. Moreover, what is needed is a weighted material that can be used as a blanket to effectively produce deep pressure therapy to an individual without limiting breathability. Additionally, what is needed is a weighted material that can be used as a blanket to effectively produce deep pressure therapy to an individual without diminishing the surface contact area of the deep pressure therapy.

### SUMMARY

As will be described in greater detail below, the disclosure describes a weighted blanket that is configured to effectuate deep pressure therapy, without the need for additional weights, such as external weights, internal weights, and the like.

In one example, a weighted material is provided. The weighted material is configured to effectuate deep pressure therapy to a person when a piece of the weighted material is used as a blanket over the person's body. A length of layered yarn is interlooped to form the piece of weighted material. The layered yarn includes an outer tube extending longitudinally from a first end to a second end. The outer tube defines a conduit extending longitudinally therethrough from the first end to the second end. A plurality of inner layers of material are disposed within the conduit and extend longitudinally from the first end to the second end. The interlooped length of yarn creates a weighted blanket that is configured and sufficiently weighted to effectuate, by itself, deep pressure therapy to a person when the blanket lies over the person's body. Various other systems and methods are also disclosed.

One general aspect includes a deep pressure therapy blanket according to claim 1.

One general aspect includes a method of constructing a piece of weighted material for a deep pressure therapy blanket according to claim 13.

An example may include a piece of weighted material, including: a length of layered yarn that is interlooped to form a piece of weighted material, where the length of layered yarn includes an outer tube extending longitudinally from a first end to a second end, the outer tube defining a conduit extending longitudinally therethrough from the first end to the second end; and a plurality of inner layers of material disposed within the conduit and extending longitudinally from the first end to the second end. The piece of weighted material also includes where the length of layered yarn, by itself, is configured and sufficiently weighted to effectuate deep pressure therapy to a person when the weighted material lies over a person's body.

### BRIEF DESCRIPTION OF THE DRAWINGS

The accompanying drawings illustrate a number of exemplary aspects and are a part of the specification. Together with the following description, these drawings demonstrate and explain various principles of the disclosure.
Figure 1 is a top view of a weighted material comprising a layered yarn that is interlooped, according to an aspect of the disclosure.
Figure 2 is a side view of a layered yarn that can be interlooped to construct the weighted material of Figure 1, according to an aspect of the disclosure.
Figure 3 is a cross-sectional view of the layered yarn of Figure 2 taken across line III-III, according to an aspect of the disclosure.
Figure 4 is a perspective view of the layered yarn of Figure 2, according to an aspect of the disclosure.
Figures 5A, 5B, 5C, 5D, and 5E show exemplary details of forming inner layers by folding an inner sheet, according to an aspect of the disclosure.
Figures 6A, 6B, and 6C show exemplary details of constructing a layered yarn, according to an aspect of the disclosure.
Figures 7A, 7B, and 7C show exemplary details of constructing a layered yarn, according to an aspect of the disclosure.
Figure 8A is a perspective view of a layered yarn being knit to construct a piece of weighted material, according to an aspect of the disclosure.
Figure 8B is a perspective view of a layered yarn being crocheted to construct a piece of weighted material, according to an aspect of the disclosure.
Figure 9 is a top view of a piece of weighted material that is constructed by knitting layered yarn, according to an aspect of the disclosure.
Figure 10 is a flowchart of an exemplary method of constructing a piece of weighted material, according to an aspect of the disclosure.

Throughout the drawings, identical reference characters and descriptions indicate similar, but not necessarily identical, elements. While the exemplary aspects described herein are susceptible to various modifications and alternative forms, specific aspects have been shown by way of example in the drawings and will be described in detail herein. The exemplary aspects described herein, however, are not intended to be limited to the particular forms disclosed.

### DETAILED DESCRIPTION OF EXEMPLARY ASPECTS

The disclosure is generally directed to a weighted material that can be used as a blanket to provide a person with deep pressure therapy, without the need for weights or components.

As used herein, the terms "providing deep pressure therapy," "bringing about deep pressure therapy," "effectuating deep pressure therapy" and/or terms similar thereto, refer to effectively causing deep pressure therapy (also known as deep pressure stimulation, deep touch pressure, etc.) in a person as herein described. More particularly, the terms "providing deep pressure therapy," "bringing about deep pressure therapy," "effectuating deep pressure therapy" and/or terms similar thereto, refer to physically contacting an individual to effectively cause the individual's nervous system activity to switch from being dominated by their sympathetic nervous system to being dominated by their parasympathetic nervous system.

An individual's autonomic nervous system (ANS) receives information from the individual's body and environment, and in response thereto, sends signals out to regulate the individual's body and organs. The ANS may include the sympathetic system, the parasympathetic system, and/or other physiological systems, which work together to help the individual physiologically respond in accordance with the information the ANS receives.

The sympathetic nervous system may often be referred to as the "alert" or "fight or flight" response that is elicited during stressful situations, emergency situations, and/or like situations. The parasympathetic nervous system, on the other hand, is responsible for regulating involuntary functions, such as heart rate, blood pressure, and/or the like and stimulating the digestive tract. It brings a sense of calm, peace, and/or the like to the mind and body. When the parasympathetic nervous system takes over, an individual's heart rate may slow, muscles may relax, circulation may improve, and/or the like. When deep pressure is correctly applied, it may relax the nervous system, causing the body to switch from running its sympathetic nervous system to its parasympathetic nervous system.

Deep touch pressure may also alter the person's hormone levels by decreasing their level of cortisol (which causes anxiety and/or other negative physiological effects) while increasing their levels of serotonin and dopamine (which help with mood regulation, relaxation, and/or other positive physiological effects). In other words, the disclosed deep touch pressure provided by the disclosed implementations may reduce anxiety and/or other negative physiological effects; and may provide positive mood regulation, relaxation, and/or other positive physiological effects.

The disclosed systems and methods include a weighted material 200 that provides a person with tactile sensory input, which provides proprioceptive input to the individual's body. The weighted material 200 is configured such that when it is administered as a blanket to cover an individual's body, it brings about deep pressure therapy (DPT), thus causing the individual's parasympathetic system to increase, their sympathetic system to decrease, and/or other beneficial responses.

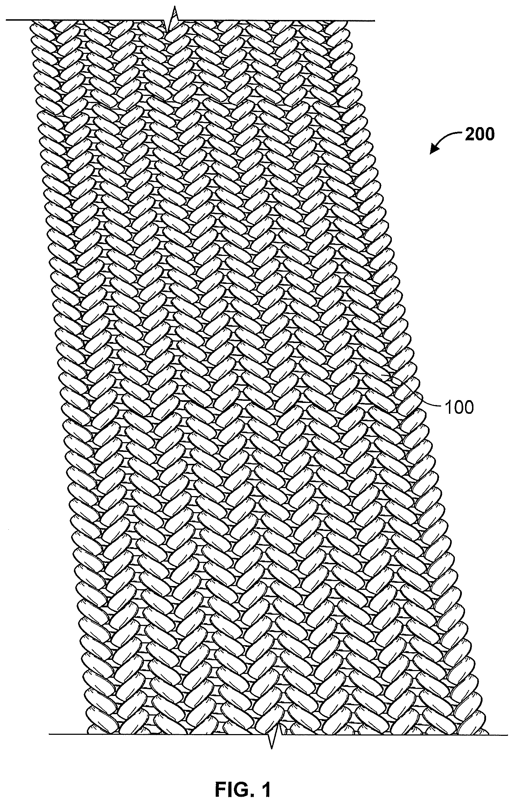

Figure 1 is a top view of a weighted material comprising a layered yarn that is interlooped, according to an aspect of the disclosure.

In particular, Figure 1 is a top view of a piece of the weighted material 200 that includes a layered yarn 100 that is interlooped (e.g., knit, crocheted, etc.). In one aspect, the layered yarn 100 that is interlooped may have a knitted construction. In one aspect, the knitted construction may be achieved by hand-knitting, machine knitting, and the like. In one or more aspects, the hand-knitting, machine knitting, and the like may be performed with or without knitting needles. In one aspect, the layered yarn 100 that is interlooped may have a crocheted construction. The construction of the layered yarn 100, together with the interlooping thereof, provides a weighted material 200 that is sufficiently heavy to provide deep pressure therapy (DPT) to a person without the need for additional weights. The weighted material 200 is configured so that the interlooped configuration of the layered yarn 100 (e.g., one layer of the interlooped configuration of the layered yarn 100), by itself and without any added weights or components, brings about deep pressure therapy (DPT) in an individual, when the weighted material 200 is employed as a blanket over the individual.

As will be described in further detail below, the disclosed weighted material 200 can be employed as a deep pressure therapy (DPT) blanket that provides many advantages that are not provided by conventional deep pressure therapy (DPT) blankets. For example, the disclosed weighted material 200 includes a layered yarn 100 that is interlooped in a pattern that creates small openings at the crossing of the loops. This allows for even airflow through the blanket, thus providing desirable breathability, and helping to regulate the person's body temperature.

The interlooping of the layered yarn 100 may create heavy knots at the cross- sections of the loops. This pattern of heavy knots, in turn, creates a pattern of pressure regions on the individual when the weighted material 200 configured as a blanket and is placed over them. This provides a highly effective deep pressure therapy blanket.

Also, the disclosed weighted material 200 offers even weight distribution. The layered yarn 100 may be evenly interlooped (e.g., knit, crochet) in equally sized, enmeshed loops, which distributes the weight substantially equally and/or evenly across the structure of the weighted material 200. The layered yarn 100 is sufficiently heavy and is fixated through the wide-looped pattern.

Thus, unlike conventional deep pressure therapy (DPT) blankets, the weight does not shift throughout the structure of the weighted material 200 when the weighted material 200 moves. This is highly advantageous, as continuously maintaining an even weight distribution across the individual is imperative to the efficacy of deep pressure therapy (DPT).

Also, the disclosed weighted material 200 provides improved body-contouring abilities. The stretchability of the interlooped configuration of the layered yarn 100 may take a natural body contouring shape and can adapt to each individual body shape, thus creating a direct and increased surface area for the weight to apply gentle and even pressure across the body. Several other benefits and advantageous may be recognized as well.

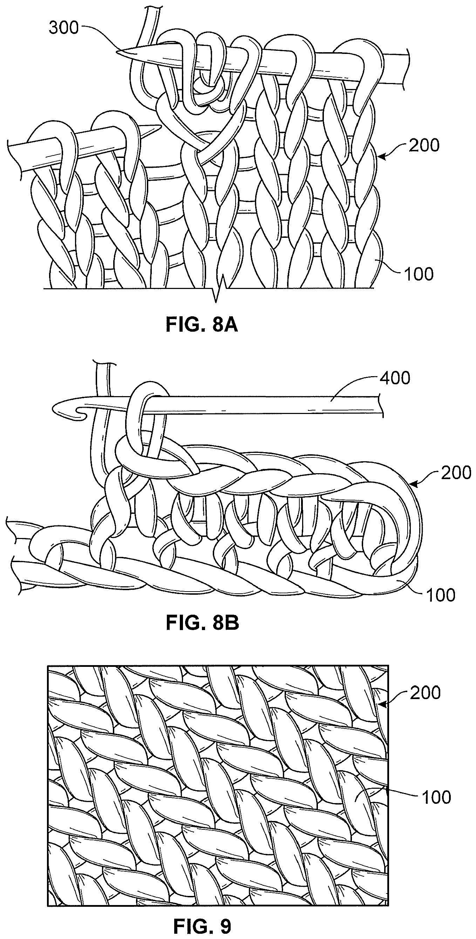

Figure 1 further shows an aspect of a weighted material 200 that is constructed by interlooping the layered yarn 100 through a knitting technique. Additionally or alternatively, other suitable techniques of interlooping the layered yarn 100 (e.g., crocheting, looping, etc.) may be implemented to form the weighted material 200 while remaining within the scope of this disclosure. For example, Figure 8B shows a piece of the weighted material 200 that is constructed by interlooping the layered yarn 100 through a crocheting technique.

The piece of the weighted material 200 shown in Figure 1 can be utilized as a blanket, for example, for covering a large percentage (e.g., a majority) of a person's body when the person is lying down. In some aspects, a blanket comprising only a single layer of the weighted material 200 provides sufficient weight to effectuate deep pressure therapy on the person. For example, the weighted material 200 may have a length, width, and height; and the layered yarn 100 may have a longitudinal length and a diameter perpendicular thereto. The height of the weighted material 200 may be equal to the diameter of the layered yarn 100. In other words, the weighted material 200 may consist of only a single layer of the interlooped configuration of the layered yarn 100 that is sufficiently weighted to effectuate deep pressure therapy (DPT) when placed over the person's body.

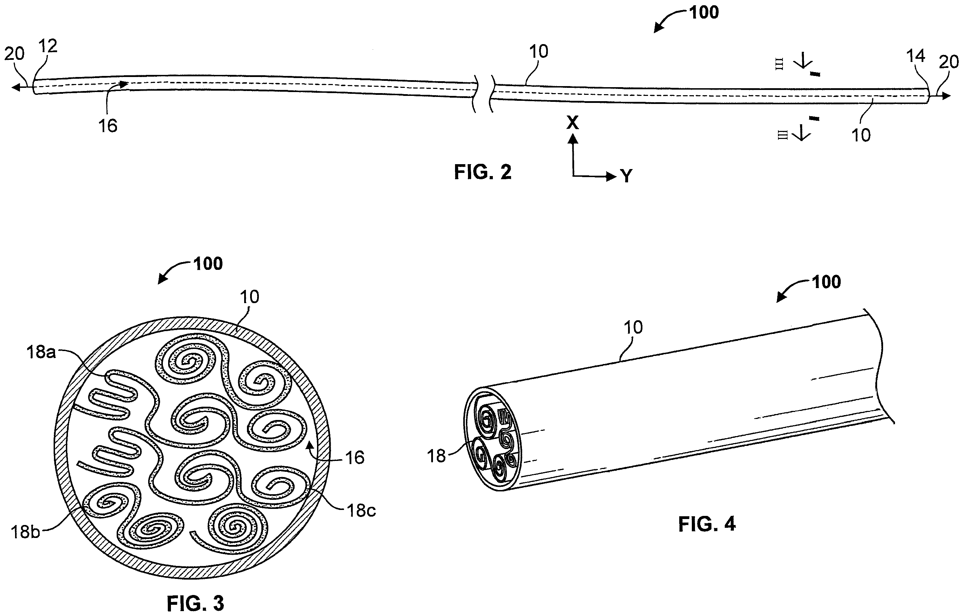

Figure 2 is a side view of a layered yarn that can be interlooped to construct the weighted material of Figure 1, according to an aspect of the disclosure; Figure 3 is a cross-sectional view of the layered yarn of Figure 2 taken across line III-III, according to an aspect of the disclosure; and Figure 4 is a perspective view of the layered yarn of Figure 2, according to an aspect of the disclosure.

In particular, Figures 2-4 are side, cross-sectional, and perspective views of a length of the layered yarn 100, according to an aspect of the disclosure. The term yarn, as used herein, refers to a continuous strand of material that can be manipulated (e.g., interlooped) to form a fabric. The layered yarn 100 may be interlooped to provide a piece of weighted material, for example, the piece of the weighted material 200 shown in Figure 1. The layered yarn 100 comprises an outer tube 10 and a plurality of inner layers 18 (18a, 18b, 18c). The outer tube 10 and the inner layers 18 may be made from textile material, further details of which are provided below with reference to Figures 3 and 4. The outer tube 10 extends longitudinally from a first end 12 to a second end 14. While Figure 2 shows an aspect in which the first end 12 and second end 14 are separated from one another, in other aspects, the length of the layered yarn 100 may comprise a loop, in which the first end 12 and the second end 14 are connected to each other.

The outer tube 10 defines a conduit 16 that extends longitudinally therethrough, from the first end 12 to the second end 14. A plurality of inner layers 18 of material are disposed within the conduit 16 and extend longitudinally from the first end 12 to the second end 14.

In one aspect, the inner layers 18 may be formed by randomly bunching up at least one inner sheet of the inner layer 18. In one aspect, the inner layers 18 may be formed by randomly clustering, bundling, grouping, and the like the at least one inner sheet of the inner layer 18. In one aspect, the inner layers 18 may be formed by folding at least one inner sheet of the inner layer 18 about at least one fold axis extending substantially longitudinally from the first end 12 to the second end 14. The inner layers 18 may be configured to provide equal weight distribution along a longitudinal length 20 of the layered yarn 100. The inner sheets of the inner layers 18 may be folded longitudinally in any suitable configuration. One or more of the inner sheets of the inner layers 18 may be folded about its respective fold axis a plurality of rotations to provide a coil shape. In some aspects, the inner sheet of the inner layers 18 may be folded about a plurality of fold axes, each of plurality of fold axes may extend longitudinally. The inner layers 18 may include any suitable number of folded inner sheets of the inner layers 18. For example, one to twenty folded inner sheets of the inner layers 18, one to sixteen folded inner sheets of the inner layers 18, one to twelve folded inner sheets of the inner layers 18, one to eight folded inner sheets of the inner layers 18, one to four folded inner sheets of the inner layers 18, two to twenty folded inner sheets of the inner layers 18, two to sixteen folded inner sheets of the inner layers 18, two to twelve folded inner sheets of the inner layers 18, two to eight folded inner sheets of the inner layers 18, or two to four folded inner sheets of the inner layers 18.

As shown in Figures 3 and 4, the inner sheet of the inner layer 18 is folded onto itself into one or more coiled or otherwise folded configuration(s). As further shown in Figures 3 and 4, the plurality of inner sheets of the inner layers 18 can each be folded in various fold arrangements. For example, Figure 3 shows an inner sheet of the inner layer 18a that is folded about one respective fold axis in a plurality of rotations to provide a coil shape. Figure 3 also shows an inner sheet of the inner layer 18b that is folded about two respective fold axes in a plurality of rotations to provide two coil shapes. The fold arrangements shown and described herein are exemplary, and the inner layers 18 may include any suitable fold arrangement while remaining within the scope of this disclosure.

The fold axis may extend substantially longitudinally along the longitudinal length 20 from the first end 12 to the second end 14, so that the inner sheet of the inner layer 18 may be folded onto itself lengthwise. The fold axis, however, does not need to be entirely straight or parallel to the longitudinal axis of the layered yarn 100. For example, the folded inner sheet of the inner layer 18 may be twisted, squished, and/or the like along the length of the layered yarn 100.

The plurality of inner layers 18 may extend uninterrupted along the longitudinal length 20 of the layered yarn 100. For example, the at least one folded inner sheet of the inner layers 18 may be uninterrupted along the longitudinal length 20 (e.g., extending uninterrupted from the first end 12 to the second end 14 of the layered yarn 100). This may provide a layered yarn 100 having a substantially homogeneous weight distribution along its length, which in turn may provide a weighted material 200 that has a substantially homogeneous weight distribution across its surface. The plurality of inner layers 18 may be disposed within the conduit 16 and fill the conduit 16 or hollow portion of the outer tube 10 by any suitable proportion. For example, the folded inner sheets of the inner layers 18 may take up 50-99% of the volume of the conduit 16. For example, the folded inner sheets of the inner layers 18 may take up 75-99% of the volume of the conduit 16. For example, the folded inner sheets of the inner layers 18 may take up 85-95% of the volume of the conduit 16. For example, the folded inner sheets of the inner layers 18 may take up to about 50% of the volume of the conduit 16. For example, the folded inner sheets of the inner layers 18 may take up to about 70% of the volume of the conduit 16. For example, the folded inner sheets of the inner layers 18 may take up to about 90% of the volume of the conduit 16. The inner layers 18 may be configured to take up enough volume within the conduit to provide sufficient weight, while allowing for sufficient air flow to flow through the layered yarn 100.

**The** outer tube 10 and the inner layers 18 may be made of any suitable textile materials, for example, knitted fabric, spun fibers, woven fibers, and/or the like. In one aspect, the outer tube 10 and/or the inner layers 18 may be made from cotton, for example, organic cotton. In one aspect, the organic cotton may be entirely 100% organic cotton. In one aspect, the outer tube 10 may include a knitted fabric that is stretchable and can be formed into a shape and may not be easily stretched out. In one aspect, the knitted fabric may be blended with a stretchable fabric such as Spandex, Lycra, elastane, or the like. In one aspect, the knitted fabric may be blended with 2% - 20% Spandex, 3% - 10% Spandex, 4% - 6% Spandex, or 5% Spandex, and the remainder of the knitted fabric may be cotton. The outer tube 10 and/or the inner layers 18 may include a material that demonstrates high stretching properties, such as elastane, to facilitate interlooping of the layered yarn 100.

In some aspects, the outer tube 10 may be made of a cotton-elastane mix, and the inner layers 18 may be made almost entirely (e.g., entirely) of cotton. In one aspect, the organic cotton may be entirely 100% organic cotton. This construction provides a layered yarn 100 that demonstrates sufficient stretchability (from the elastane material in the outer tube 10) to facilitate interlooping of the layered yarn 100 and body contouring of the weighted material 200, while also maintaining sufficient rigidity to provide steady weight distribution of the weighted material 200 configured as a blanket. In one aspect, these characteristics may be a result of the cotton material in the outer tube 10 and/or the cotton material of the inner layers 18.

The weighted material 200 may have any suitable dimensions to be employed as a blanket over a person laying down to bring about deep pressure therapy (DPT). For example, the weighted material 200 may have a length between 12.7 and 228.6 cm (40 and 90 inches), between 127 and 203.2 cm (50 and 80 inches), or between 165.1 and 190.5 cm (65 and 75 inches). The weighted material 200 may have a width between 63.5 and 203.2 cm (25 and 80 inches), between 88.9 and 177.8 cm (35 and 70 inches), or between 114.3 and 139.7 cm (45 and 55 inches).

In some aspects, a length of the layered yarn 100 is between 100 and 300 meters in length, between 200 and 300 meters in length, between 225 and 300 meters in length, or between 225 and 275 meters in length. In some aspects, a length of the layered yarn 100 is generally equal to a length of a full roll of fabric.

In some aspects, the weighted material 200 is about 121.92 cm (48 inches) wide, 182.88 cm (72 inches) long, and 5.08 cm (2 inches) high (thick), and is made of a length of the layered yarn 100 having an interlooped configuration that is about 250 meters long and that has a diameter of about 5.08 cm (2 inches).

The weighted material 200 may be configured to weigh between 2.27 and 20.42 kg (5 and 45 lb.), between 3.17 and 18.14 kg (7 and 40 lb.), or between 4.54 and 15.87 kg (10 and 35 lb.). The weighted material 200 may weigh between 10% and 20% of a person's body weight, and the weighted material 200 may be configured to bring about deep pressure therapy (DPT) for a person weighing between, for example, 15.87 kg (35 lb.), and 400 lb. In some aspects, the piece of the weighted material 200 has dimensions of about 121.92 x 182.88 x 5.08 cm (48 x 72 x 2 inches), weights between 9.07 and 15.87 kg (20 and 35 lb.), and is constructed from a length of interlooped layer yarn that is about 250 meters long and has a diameter of about 5.08 cm (2 inches).

The layered yarn 100 may have a diameter between 2.54 and 12.7 cm ( 1 and 5 inches), For example, the layered yarn 100 may have a diameter between 3.81 and 7.62 cm (1.5 and 3 inches). For example, the layered yarn 100 may have a diameter between 4.445 and 5.715 cm (1.75 and 2.25 inches). In some aspects, the layered yarn 100 has a diameter of about 5.08 cm (2 inches). In some aspects, the plurality of inner layers 18 comprise folded inner sheets that have a length that is substantially the same as the length of the hollow portion of the outer tube 10, and the inner sheets of the inner layers 18 have a width that is greater than the diameter of the hollow portion of the outer tube 10.

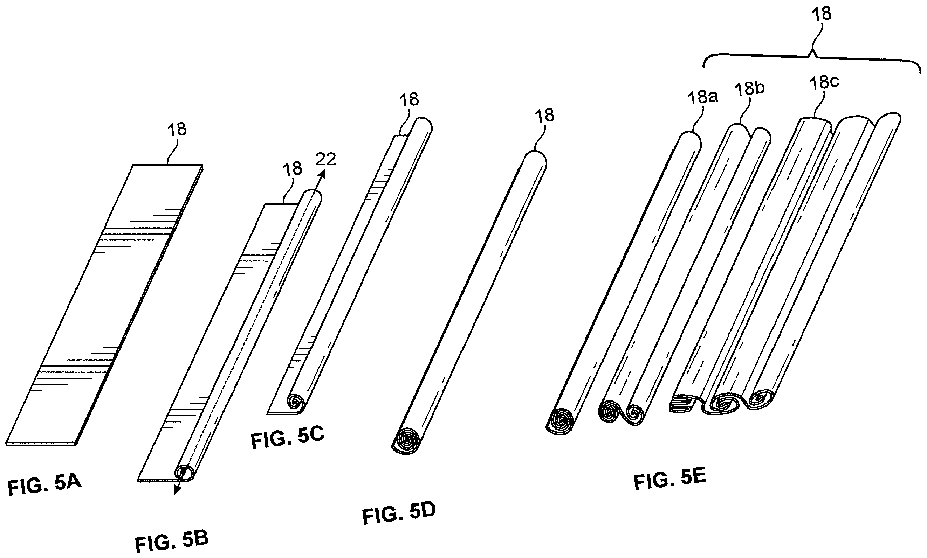

Figures 5A, 5B, 5C, 5D, and 5E show exemplary details of forming inner layers by folding an inner sheet, according to an aspect of the disclosure.

In particular, Figures 5A-5E show a schematic representation of forming the inner layers 18 by folding an inner sheet of the inner layer 18, according to an aspect of the disclosure. Figure 5A shows an inner sheet of the inner layer 18 that is in a substantially flat configuration. The inner sheet of the inner layer 18 shown in Figure 5A may be formed of a larger configuration of the inner sheet of the inner layer 18 that is folded along one or more fold lines to provide the substantially flat configuration shown in Figure 5A.

Figures 5B and 5C show the inner sheet of the inner layer 18 of Figure 5A as it is being rolled along the longitudinally extending fold axis 22. Figure 5D shows the inner sheet of the inner layer 18 of Figures 5A-5C that is rolled along the longitudinally extending fold axis 22. Figure 5E shows the inner sheet of the inner layer 18a of Figures 5A-5D, together with additional inner sheets of the inner layers 18b, 18c that are each folded along their respective longitudinally extending fold axes. The inner sheets of the inner layers 18 can be arranged within the hollow portion of the outer tube 10 to form the layered yarn 100. In one aspect, the inner sheets of the inner layers 18 can be pulled into the hollow portion of the outer tube 10 to form the layered yarn 100. In one aspect, the inner sheets of the inner layers 18 can be inserted into the hollow portion of the outer tube 10 to form the layered yarn 100. In one aspect, the inner sheets of the inner layers 18 can be drawn into the hollow portion of the outer tube 10 to form the layered yarn 100.

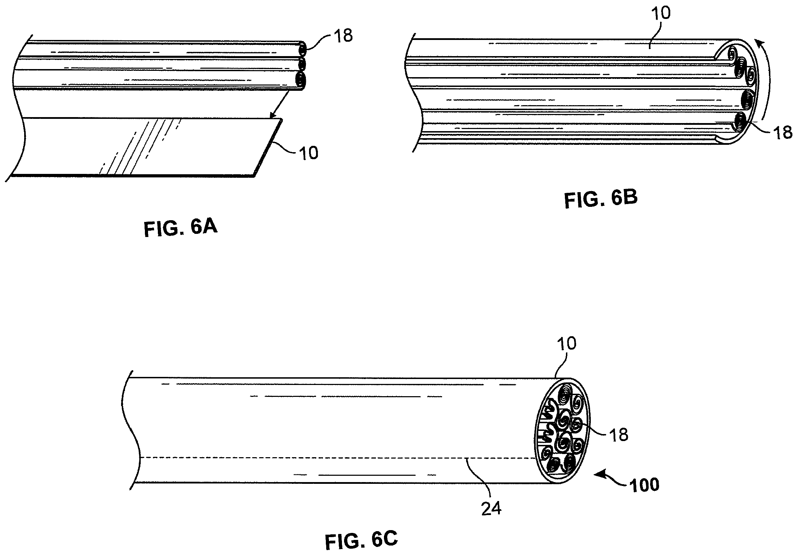

Figures 6A, 6B, and 6C show exemplary details of constructing a layered yarn, according to an aspect of the disclosure.

In particular, Figures 6A-6C show a schematic representation of constructing a layered yarn 100, according to an aspect of the disclosure. Figure 6A shows the outer tube 10 that is arranged as a sheet (e.g., sheet of fabric) before it is rolled into the hollow portion of the outer tube 10. Also shown in Figure 6A is the at least one folded inner layer 18 as it is being arranged on top of a sheet of the outer tube 10. In other aspects, the inner layer 18 may be randomly pulled, drawn, inserted, or the like into the outer tube 10. Figure 6B shows the at least one folded inner layer 18 disposed on the outer tube 10, and the outer tube 10 being rolled into the hollow portion of the outer tube 10. Figure 6C shows the hollow portion of the outer tube 10 rolled around the plurality of inner layers 18 to form the layered yarn 100. The outer tube 10 may be attached at a seam 24 by any suitable means (e.g., stitching, adhesion, friction, hook-and-loop, etc.) to secure the hollow portion of the outer tube 10. In one aspect, the outer tube 10 may be attached at the seam 24 by stitching. In one aspect, the outer tube 10 may be attached at the seam 24 by stitching and thereafter an orientation of the tube 10 may be reversed to place the seam 24 within the outer tube 10. In one aspect, the outer tube 10 may be attached at the seam 24 by adhesion. In one aspect, the outer tube 10 may be attached at the seam 24 by friction. In one aspect, the outer tube 10 may be attached at the seam 24 by hook-and-loop. The hollow portion of the outer tube 10 may be attached to itself at the first end 12 and the second end 14 to close the conduit 16.

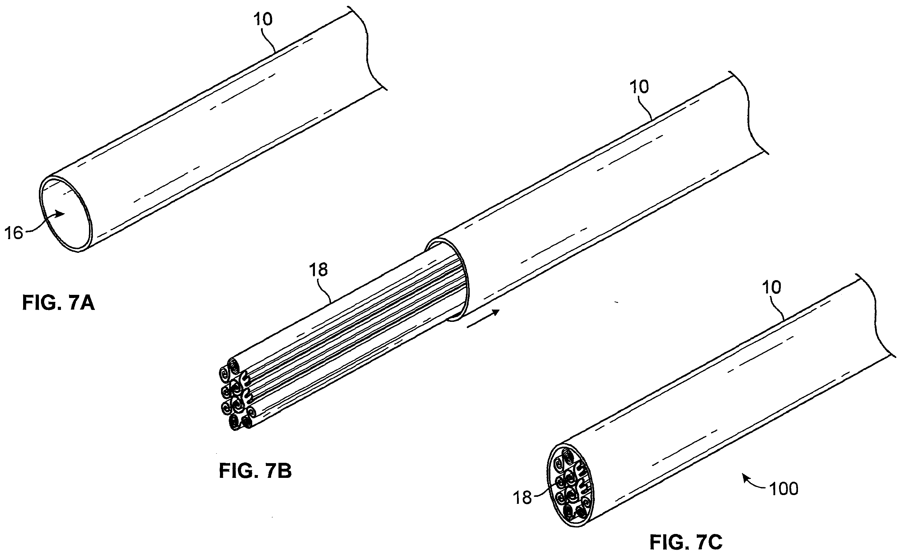

Figures 7A, 7B, and 7C show exemplary details of constructing a layered yarn, according to an aspect of the disclosure.

In particular, Figures 7A-7C show a schematic representation of constructing a layered yarn 100, according to an aspect of the disclosure. Figure 7A shows the outer tube 10 that is hollow, with conduit 16 extending longitudinally therethrough. In some aspects, the outer tube 10 is formed by rolling an outer layer sheet and securing it at a seam into a tube shape (e.g., by attaching the rolled sheet by suitable means (e.g., stitching, adhesion, friction, hook-and-loop, etc.) similarly to the seam 24 shown in Figure 6C. In some aspects, the outer tube 10 is formed as a seamless tubular structure. For example, the outer tube 10 may be formed by interlocking fibers to form the tubular configuration. Figure 7B shows the plurality of inner layers 18 being inserted into the conduit 16 of the hollow portion of the outer tube 10. Figure 7C shows the plurality of inner layers 18 that are arranged inside the hollow portion of the outer tube 10 to provide the layered yarn 100. The hollow portion of the outer tube 10 may be attached to itself at the first end 12 and the second end 14 to close the conduit 16.

In some aspects, the plurality of inner layers 18 may be attached together by any suitable means to maintain, for example, the folded configuration. For example, the at least one folded inner sheets of the inner layers 18 may be stitched and/or adhered to itself. Additionally or alternatively, the plurality of inner layers 18 may be attached to at least a portion of the inner tubular wall of the hollow portion of the outer tube 10 by any suitable means. For example, the at least one folded inner sheet of the inner layer 18 may be attached to the inner tubular wall of the hollow portion of the outer tube 10 by way of stitching, adhesion (e.g., glue), friction, hook-and-loop, etc.

Figure 8A is a perspective view of a layered yarn being knit to construct a piece of weighted material, according to an aspect of the disclosure.

Figure 9 is a top view of a piece of weighted material that is constructed by knitting layered yarn, according to an aspect of the disclosure.

Figure 8A is a perspective view of a layered yarn 100 that is being knit to construct a piece of the weighted material 200, according to an aspect of the disclosure. Figure 9 is a top view of a piece of the weighted material 200 that is constructed by knitting the layered yarn 100 (e.g., as shown in Figure 8A), according to an aspect of the disclosure. The layered yarn 100 may be knit using a pair of knitting needles 300. The knitting may be performed manually by a user and/or using an automated machine. The knitting may comprise intermeshing loops of the layered yarn 100 in a number of consecutive rows. As each row progresses, a newly generated loop may be pulled through one or more loops from the prior row, creating a dense pattern.

Figure 8B is a perspective view of a layered yarn being crocheted to construct a piece of weighted material, according to an aspect of the disclosure.

In particular, Figure 8B is a perspective view of a strand of the layered yarn 100 being crocheted to construct a piece of the weighted material 200, according to an aspect of the disclosure. The layered yarn 100 may be crocheted using a pair of crocheting needles 400, as shown. The crocheting may be performed manually by a user and/or using an automated machine. The crocheting may comprise intermeshing loops of the layered yarn 100 in a number of consecutive rows.

As shown in Figures 8A and 8B, the weighted material 200 comprises layered yarn 100 that is interlooped in a pattern that creates small openings at the crossing of the loops. This allows for even airflow through the blanket, thus providing desirable breathability and helping to regulate the individual's body temperature. The interlooping of the layered yarn 100 (e.g., knitting as shown in Figure 8A, crocheting as shown in Figure 8B), together with the construction of the layered yarn 100, creates a piece of the weighted material 200 that is dense enough to provide deep pressure therapy (DPT) for a person. For example, the piece of the weighted material 200 can have dimensions, of about 48 x 72 x 2 inches and can weigh between 20 and 35 lb. The interlooped configuration of the weighted material 200 is both sufficiently wide-looped and dense to effectuate deep pressure therapy (DPT), while also providing superior breathability, even and continuous weight distribution, and natural body contouring capabilities.

In one aspect, the interlooped configuration of the weighted material 200 comprises substantially a textile material. In one aspect, the interlooped configuration of the weighted material 200 comprises substantially a fabric material. In one aspect, the interlooped configuration of the weighted material 200 comprises substantially a fiber material. In one aspect, the interlooped configuration of the weighted material 200 comprises substantially a cotton material.

In one aspect, the interlooped configuration of the weighted material 200 comprises 90% - 100% by weight of a textile material. In one aspect, the interlooped configuration of the weighted material 200 comprises 90% - 100% by weight of a fabric material. In one aspect, the interlooped configuration of the weighted material 200 comprises 90% - 100% by weight of a fiber material. In one aspect, the interlooped configuration of the weighted material 200 comprises 90% - 100% by weight of a cotton material.

In one aspect, the interlooped configuration of the weighted material 200 entirely includes a textile material. In one aspect, the interlooped configuration of the weighted material 200 entirely includes a fabric material. In one aspect, the interlooped configuration of the weighted material 200 entirely includes a fiber material. In one aspect, the interlooped configuration of the weighted material 200 entirely includes a cotton material.

The interlooping of the layered yarn 100 creates heavy knots at the cross- sections of the loops. This pattern of heavy knots, in turn, creates a pattern of pressure regions on the individual when the weighted material 200 configured as a blanket and is placed over them. This provides a highly effective deep pressure therapy (DPT) blanket.

Figures 8A and 8B show formation of a single layer of interlooped configuration of the layered yarn 100. For example, the thickness of the weighted material 200 is equal to the diameter of the layered yarn 100. In some aspects, the layered yarn 100 is sufficiently heavy, and the layered yarn 100 is interlooped such that only a single layer of the interlooped configuration of the layered yarn 100 creates a piece of the weighted material 200 that, when employed as a blanket over a person, brings about deep pressure therapy (DPT) to the person.

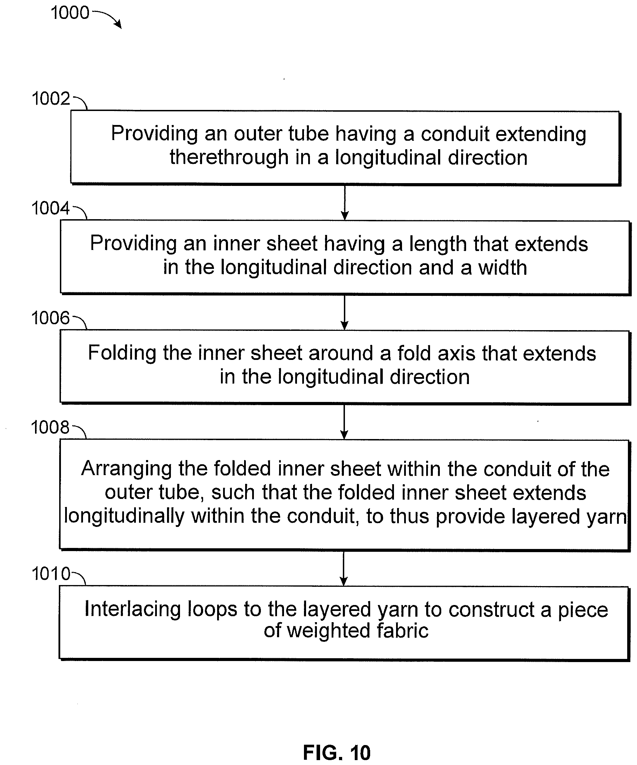

Figure 10 is a flowchart of an exemplary method of constructing a piece of weighted material, according to an aspect of the disclosure.

In particular, Figure 10 is a flowchart of a method 1000 of constructing a piece of weighted material 200, according to an aspect of the disclosure. In steps 1002-1008, a layered yarn 100 is constructed. At step 1002, an outer tube 10 is provided. The outer tube includes a conduit 16 extending therethrough in a longitudinal direction. At steps 1004 and 1006, an inner sheet is provided and folded around a fold axis, which extends in a longitudinal direction. At step 1008, the folded inner sheet is arranged within the conduit 16, such that the folded inner sheet extends longitudinally within the conduit 16. At step 1010, the constructed layered yarn is interlooped to construct the weighted material 200 or piece of deep pressure therapy (DPT) material.

The weighted material 200 of Figure 1 is employed as a blanket over a person's body (e.g., a majority of the person's body). The weighted material 200 configured as a blanket may be configured to provide sufficient air circulation through the weighted material 200 configured as a blanket to regulate the body temperature of the person. For example, the weighted material 200 configured as a blanket may be configured to provide 18-26% air flow therethrough (e.g., from the air above the weighted material 200 configured as a blanket to the person laying beneath the weighted material 200 configured as a blanket).

The weighted material 200 may have beneficial air flow qualities that may be provided by the interlooping of the layered yarn 100 (allowing air to circulate around the layered yarn 100) as well as the construction of the layered yarn 100 (allowing air to circulate through the layered yarn 100). For example, as described above with reference to Figures 8A and 8B, the weighted material 200 may be created by an interlooped pattern that creates small openings at the crossing of the loops, and between the loops of layered yarn 100. Thus, air flow may travel from the top and to the bottom of the weighted material 200 through the small openings (the air flowing around the layered yarn 100). Also, the layered yarn 100 may be configured to allow air to circulate through the yarn itself. For example, the configuration of the hollow portion of the outer tube 10 and the plurality of the inner layers 18 provide empty spaces within the conduit 16. Air is thus allowed to circulate along the empty spaces and through the conduit 16 (the air flowing through the layered yarn 100). In addition to providing superior air circulation, the construction of the weighted material 200 also provides well-balanced moisture and heat transfer capabilities.

The layered yarn 100 may be configured to have a substantially homogenous weight and density along its longitudinal length 20. This provides many advantages over strands of material that comprise bunched up filler material. For example, the bunched up filler material creates inconsistent weight distribution, which greatly impairs even weight distribution of the weighted blanket. The layered yarn 100, however, is configured to maintain weight distribution through the interlooping of the layered yarn 100 and the movement and use of the weighted material 200 configured as a blanket.

While the outer tube 10 and inner layers 18 are shown and described as being separate components, in some aspects, the outer tube 10 and at least one of the inner layers 18 are integrally formed. In some aspects, the inner layers 18 are attached (e.g., at a seam along the longitudinal length 20 of the layered yarn 100), so the outer periphery of the attached inner layers 18 forms the outer construction of the yarn (e.g., without a separate outer tube 10).

While this disclosure describes using the piece of the weighted material 200 to provide deep pressure therapy (DPT), it should be well understood that the weighted material 200 may be used for other purposes, in addition to or as an alternate to deep pressure therapy (DPT). Also, while the disclosure describes employing the piece of the weighted material 200 as a blanket to lay over an individual, it should be well understood that the weighted material 200 may be employed in other suitable ways. Further, while this disclosure describes laying the weighted material 200 over a person, it should be well understood that the weighted material 200 may be laid over other suitable kinds of animals to provide them with deep pressure therapy (DPT).

Unless otherwise noted, the terms "connected to" and "coupled to" (and their derivatives), as used in the specification and claims, are to be construed as permitting both direct and indirect (i.e., via other elements or components) connection. In addition, the terms "a" or "an," as used in the specification and claims, are to be construed as meaning "at least one of." Finally, for ease of use, the terms "including" and "having" (and their derivatives), as used in the specification and claims, are interchangeable with and have the same meaning as the word "comprising."

It will be understood that when an element such as a layer or region is referred to as being "on" or extending "onto" another element, it can be directly on or extend directly onto the other element or intervening elements may also be present. In contrast, when an element is referred to as being "directly on" or extending "directly onto" another element, there are no intervening elements present. It will also be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present.

Relative terms such as "below" or "above" or "upper" or "lower" or "top" or "bottom" may be used herein to describe a relationship of one element, layer or region to another element, layer or region as illustrated in the figures. It will be understood that these terms are intended to encompass different orientations of the device in addition to the orientation depicted in the figures.

While the disclosure has been described in terms of exemplary aspects, those skilled in the art will recognize that the disclosure can be practiced with modifications in the scope of the appended claims. These examples given above are merely illustrative and are not meant to be an exhaustive list of all possible designs, aspects, applications or modifications of the disclosure.

In the drawings and specification, there have been disclosed typical aspects of the disclosure and, although specific terms are employed, they are used in a generic and descriptive sense only and not for purposes of limitation, the scope of the invention being set forth in the following claims.

## Claims

1. A deep pressure therapy blanket (200), comprising a piece of weighted material, the piece of weighted material comprising:
a length of layered yarn (100) that is interlooped to form the piece of weighted material, wherein the length of layered yarn comprises:
an outer tube (10) extending longitudinally from a first end to a second end, the outer tube defining a conduit (16) extending longitudinally therethrough from the first end to the second end, the outer tube being formed of a rolled outer layer sheet (10); and
a plurality of inner layers of material (18) disposed within the conduit (16) and extending longitudinally from the first end to the second end,
wherein the length of layered yarn (100), by itself, is configured and sufficiently weighted to effectuate deep pressure therapy to a person when the deep pressure therapy blanket lies over a person's body and wherein the piece of weighted material is configured to weigh between 2.27 kg and 20.41 kg (5 pounds and 45 pounds) inclusive.

2. The deep pressure therapy blanket of claim 1, wherein:
the outer tube (10) has a diameter extending perpendicular to a longitudinal direction;
the deep pressure therapy blanket (200) has a length, a width, and a height; and
the height of the deep pressure therapy blanket is equal to the diameter of the outer tube.

3. The deep pressure therapy blanket of claim 1, wherein the plurality of inner layers (18) comprises a folded inner sheet, and wherein the folded inner sheet is folded about a fold axis that extends longitudinally from the first end to the second end.

4. The deep pressure therapy blanket of claim 1,
wherein the length of layered yarn (100) is interlooped with at least one of the following: a knitting construction or a crocheting construction.

5. The deep pressure therapy blanket of claim 1, wherein the length of layered yarn is interlooped in a pattern that creates openings for airflow through the deep pressure therapy blanket.

6. The deep pressure therapy blanket of claim 1, wherein the length of layered yarn (100) has a substantially homogenous weight along its longitudinal length.

7. The deep pressure therapy blanket (200) according to any preceding claim, wherein the piece of weighted material comprises a fiber material.

8. The deep pressure therapy blanket (200) according to any of claims 1-6, wherein the outer tube and the plurality of inner layers comprise a textile material selected from a group comprising knitted fabric, spun fibers and woven fibers.

9. The deep pressure therapy blanket (200) according to any of claim 1-6, wherein the outer tube and/or the plurality of inner layers comprise a stretchable material and optionally wherein the stretchable material is elastane.

10. The deep pressure therapy blanket (200) according to any preceding claim, wherein:
(i) the blanket has a length between 127 cm and 203 cm (50 inches and 80 inches) inclusive; or
(ii) the blanket has a width between 88.9 cm to 177.8 cm (35 inches to 70 inches) inches.

11. The deep pressure therapy blanket (200) according to any preceding claim, wherein the length of layered yarn (100) that is interlooped to form the piece of weighted material is configured to form loops and knots at cross-sections of the loops to create a pattern of pressure regions on the person when the deep pressure therapy blanket lies over a person's body.

12. The deep pressure therapy blanket (200) according to any preceding claim, wherein the piece of weighted material is configured to weigh between:
(i) 3.17 kg and 18.14 kg (7 pounds and 40 pounds) inclusive;
(ii) 4.54 kg and 15.88 kg (10 pounds and 35 pounds) inclusive; or
(iii) 9.07 kg and 15.87 kg (20 pounds and 35 pounds) inclusive, having a density between 0.08 g/cm³ and 0.14 g/cm³.

13. A method of constructing a piece of weighted material for a deep pressure therapy blanket (200), the method comprising:
constructing a length of layered yarn (100) by:
creating an outer tube (10) extending longitudinally from a first end to a second end, the outer tube (10) defining a conduit extending longitudinally therethrough from the first end to the second end, the outer tube (10) being formed by rolling an outer layer sheet;
arranging a plurality of inner layers (18) within the conduit (16), each of the plurality of inner layers extending longitudinally from the first end to the second end; and
interlooping the length of layered yarn (100) to construct the piece of weighted material,
wherein the length of layered yarn, by itself, is configured and sufficiently weighted to effectuate deep pressure therapy to a person when the deep pressure therapy blanket lies over a person's body and wherein the piece of weighted material is configured to weigh between 2.27 kg and 20.41 kg (5 pounds and 45 pounds) inclusive.

14. The method of claim 13, wherein:
the outer tube (10) has a diameter extending perpendicular to a longitudinal direction
the piece of weighted material has a length, a width, and a height; and
the height of the deep pressure therapy blanket is equal to the diameter of the outer tube.

15. The method of claim 13, comprising:
folding an inner sheet about a fold axis that extends longitudinally from the first end to the second end to create at least one of the plurality of inner layers.

## Patentansprüche

1. Tiefdrucktherapiedecke (200), umfassend ein Stück gewichtetes Material, das Stück gewichtetes Material umfassend:
eine Länge aus geschichtetem Garn (100), das ineinander verschlungen ist, so dass das Stück aus gewichtetem Material gebildet wird, wobei die Länge aus geschichtetem Garn umfasst:
eine äußere Röhre (10), die sich in Längsrichtung von einem ersten Ende zu einem zweiten Ende erstreckt, wobei die äußere Röhre einen Kanal (16) definiert, der sich in Längsrichtung vom ersten Ende zum zweiten Ende durch die Röhre erstreckt, wobei die äußere Röhre aus einer gerollten äußeren Schichtbahn (10) gebildet ist, und
eine Vielzahl innerer Materialschichten (18), die innerhalb des Kanals (16) angeordnet sind und sich in Längsrichtung vom ersten Ende zum zweiten Ende erstrecken,
wobei die Länge des geschichteten Garns (100) selbst so beschaffen und ausreichend gewichtet ist, dass eine Tiefdrucktherapie bei einer Person durchgeführt werden kann, wenn die Tiefdrucktherapiedecke über dem Körper einer Person liegt, und wobei das Stück aus gewichtetem Material so beschaffen ist, dass es zwischen 2,27 kg und 20,41 kg (5 Pfund und 45 Pfund) inklusive wiegt.

2. Tiefdrucktherapiedecke nach Anspruch 1, wobei:
die äußere Röhre (10) einen Durchmesser aufweist, der sich senkrecht zu einer Längsrichtung erstreckt,
die Tiefdrucktherapiedecke (200) eine Länge, eine Breite und eine Höhe aufweist und
die Höhe der Tiefdrucktherapiedecke dem Durchmesser der äußeren Röhre entspricht.

3. Tiefdrucktherapiedecke nach Anspruch 1, wobei die Vielzahl von inneren Schichten (18) eine gefaltete innere Bahn umfasst und wobei die gefaltete innere Bahn um eine Faltachse gefaltet ist, die sich in Längsrichtung vom ersten Ende zum zweiten Ende erstreckt.

4. Tiefdrucktherapiedecke nach Anspruch 1, wobei die Länge des geschichteten Garns (100) mit mindestens einem des Folgendem verflochten ist: einer Strickkonstruktion oder einer Häkelkonstruktion.

5. Tiefdrucktherapiedecke nach Anspruch 1, wobei die Länge des geschichteten Garns in einem Muster miteinander verflochten ist, das Öffnungen für den Luftstrom durch die Tiefdrucktherapiedecke schafft.

6. Tiefdrucktherapiedecke nach Anspruch 1, wobei die Länge des geschichteten Garns (100) über seine Längserstreckung ein im Wesentlichen homogenes Gewicht aufweist.

7. Tiefdrucktherapiedecke (200) nach einem der vorhergehenden Ansprüche, wobei das Stück gewichtetes Material ein Fasermaterial umfasst.

8. Tiefdrucktherapiedecke (200) nach einem der Ansprüche 1 bis 6, wobei die äußere Röhre und die Vielzahl von inneren Schichten ein Textilmaterial umfassen, das aus einer Gruppe ausgewählt ist, die Maschenware, gesponnene Fasern und gewebte Fasern umfasst.

9. Tiefdrucktherapiedecke (200) nach einem der Ansprüche 1 bis 6, wobei die äußere Röhre und/oder die Vielzahl von inneren Schichten ein dehnbares Material umfassen und wobei das dehnbare Material optional Elasthan ist.

10. Tiefdrucktherapiedecke (200) nach einem der vorhergehenden Ansprüche, wobei:
(i) die Decke eine Länge zwischen 127 cm und 203 cm (50 Zoll und 80 Zoll) inklusive aufweist oder
(ii) die Decke eine Breite zwischen 88,9 cm und 177,8 cm (35 Zoll bis 70 Zoll) aufweist.

11. Tiefdrucktherapiedecke (200) nach einem der vorhergehenden Ansprüche, wobei die Länge des geschichteten Garns (100), das verschlungen ist, so dass das Stück gewichtetes Material gebildet wird, so beschaffen ist, dass Schlaufen und Knoten an den Querschnitten der Schlaufen gebildet werden, so dass ein Muster von Druckregionen auf die Person erzeugt werden, wenn die Tiefendrucktherapiedecke über dem Körper einer Person liegt.

12. Tiefdrucktherapiedecke (200) nach einem der vorhergehenden Ansprüche, wobei das Stück gewichtetes Material so beschaffen ist, dass es wiegt zwischen:
(i) 3,17 kg und 18,14 kg (7 Pfund und 40 Pfund) inklusive,
(ii) 4,54 kg und 15,88 kg (10 Pfund und 35 Pfund) inklusive oder
(iii) 9,07 kg und 15,87 kg (20 Pfund und 35 Pfund) inklusive, mit einer Dichte zwischen 0,08 g/cm^{®} und 0,14 g/cm³.

13. Verfahren zum Anfertigen eines Stücks gewichteten Materials für eine Tiefdrucktherapiedecke (200), das Verfahren umfassend:
Anfertigen einer Länge aus geschichtetem Garn (100) durch:
Erzeugen einer äußeren Röhre (10), die sich in Längsrichtung von einem ersten Ende zu einem zweiten Ende erstreckt, wobei die äußere Röhre (10) einen Kanal definiert, der sich in Längsrichtung vom ersten Ende zum zweiten Ende dort hindurch erstreckt, wobei die äußere Röhre (10) durch Rollen einer äußeren Schichtbahn gebildet wird,
Anordnen einer Vielzahl von inneren Schichten (18) innerhalb des Kanals (16), wobei sich jede der Vielzahl von inneren Schichten in Längsrichtung vom ersten Ende zum zweiten Ende erstreckt, und
Verflechten der Länge des geschichteten Garns (100), so dass das Stück aus gewichtetem Material gebildet wird,
wobei die Länge des geschichteten Garns selbst so beschaffen und ausreichend gewichtet ist, dass eine Tiefdrucktherapie bei einer Person durchgeführt werden kann, wenn die Tiefdrucktherapiedecke über dem Körper einer Person liegt, und wobei das Stück gewichtetes Material so beschaffen ist, dass es zwischen 2,27 kg und 20,41 kg (5 Pfund und 45 Pfund) inklusive wiegt.

14. Verfahren nach Anspruch 13, wobei:
die äußere Röhre (10) einen Durchmesser aufweist, der sich senkrecht zu einer Längsrichtung erstreckt,
das Stück gewichtetes Material eine Länge, eine Breite und eine Höhe aufweist und
die Höhe der Tiefdrucktherapiedecke dem Durchmesser der äußeren Röhre entspricht.

15. Verfahren nach Anspruch 13, umfassend:
Falten einer inneren Bahn um eine Faltachse, die sich in Längsrichtung vom ersten Ende zum zweiten Ende erstreckt, so dass mindestens eine der Vielzahl von inneren Schichten erzeugt wird.

## Revendications

1. Couverture de thérapie par pression profonde (200), comprenant une pièce de matériau lesté, la pièce de matériau lesté comprenant :
une longueur de fil en couches (100) qui est entremaillée pour former la pièce de matériau lesté, dans laquelle le longueur de fil en couches comprend :
un tube externe (10) s'étendant longitudinalement d'une première extrémité à une deuxième extrémité, le tube externe définissant un conduit (16) s'étendant longitudinalement à travers ce dernier de la première extrémité à la deuxième extrémité, le tube externe étant formé avec une feuille de couche externe laminée (10) ; et
une pluralité de couches internes de matériau (18) disposée à l'intérieur du conduit (16) et s'étendant longitudinalement de la première extrémité à la deuxième extrémité,
dans laquelle la longueur de fil en couches (100), d'elle-même, est configurée et suffisamment lestée pour effectuer la thérapie par pression profonde sur une personne lorsque la couverture de thérapie par pression profonde est sur le corps d'une personne et dans laquelle la pièce de matériau lesté est configurée pour peser entre 2,27 kg et 20,41 kg (5 livres et 45 livres) y compris.

2. Couverture de thérapie par pression profonde selon la revendication 1, dans laquelle :
le tube externe (10) a un diamètre s'étendant perpendiculairement à une direction longitudinale ;
la couverture de thérapie par pression profonde (200) a une longueur, une largeur et une hauteur ; et
la hauteur de la couverture de thérapie par pression profonde est égale au diamètre du tube externe.

3. Couverture de thérapie par pression profonde selon la revendication 1, dans laquelle la pluralité de couches internes (18) comprend une feuille interne pliée, et dans laquelle la feuille interne pliée est pliée autour d'un axe de pliage qui s'étend longitudinalement de la première extrémité à la deuxième extrémité.

4. Couverture de thérapie par pression profonde selon la revendication 1,
dans laquelle la longueur du fil en couches (100) est entremaillée avec au moins l'une des constructions suivantes : une construction de tricotage ou une construction de crochetage.

5. Couverture de thérapie par pression profonde selon la revendication 1, dans laquelle la longueur du fil en couches est entremaillée selon un motif qui crée des ouvertures ou l'écoulement d'air à travers la couverture de thérapie par pression profonde.

6. Couverture de thérapie par pression profonde selon la revendication 1, dans laquelle la longueur du fil en couches (100) a un poids sensiblement homogène le long de sa longueur longitudinale.

7. Couverture de thérapie par pression profonde (200) selon l'une quelconque des revendications précédentes, dans laquelle la pièce de matériau lesté comprend un matériau fibreux.

8. Couverture de thérapie par pression profonde (200) selon l'une quelconque des revendications 1 à 6, dans laquelle le tube externe et la pluralité de couches internes comprennent un matériau textile sélectionné dans un groupe comprenant le tissu tricoté, les fibres filées et les fibres tissées.

9. Couverture de thérapie par pression profonde (200) selon l'une quelconque des revendications 1 à 6, dans laquelle le tube externe et/ou la pluralité de couches internes comprennent un matériau extensible et facultativement dans laquelle le matériau extensible est de l'élastane.

10. Couverture de thérapie par pression profonde (200) selon l'une quelconque des revendications précédentes, dans laquelle :
(i) la couverture a une longueur comprise entre 127 cm et 203 cm (50 pouces et 80 pouces) y compris ; ou bien
(ii) la couverture a une largeur comprise entre 88,9 cm à 177,8 cm (35 pouces à 70 pouces).

11. Couverture de thérapie par pression profonde (200) selon l'une quelconque des revendications précédentes, dans laquelle la longueur du fil en couches (100) qui est entremaillée pour former la pièce de matériau lesté est configurée pour former des boucles et des noeuds au niveau des sections transversales des boucles afin de créer un motif de régions de pression sur la personne lorsque la couverture de thérapie par pression profonde est sur le corps d'une personne.

12. Couverture de thérapie par pression profonde (200) selon l'une quelconque des revendications précédentes, dans laquelle la pièce de matériau lesté est configurée pour peser entre :
(i) 3,17 kg et 18,14 kg (7 livres et 40 livres) y compris ;
(ii) 4,54 kg et 15,88 kg (10 livres et 35 livres) y compris ; ou bien
(iii) 9,07 kg et 15,87 kg (20 livres et 35 livres) y compris, ayant une densité comprise entre 0,08 g/cm³ et 0,14 g/cm³.

13. Procédé pour construire une pièce de matériau lesté pour une couverture de thérapie par pression profonde (200), le procédé comprenant le fait de :
construire une longueur de fil en couches (100) en :
créant un tube externe (10) s'étendant longitudinalement d'une première extrémité à une deuxième extrémité, le tube externe (10) définissant un conduit s'étendant longitudinalement à travers ce dernier de la première extrémité à la deuxième extrémité, le tube externe (10) étant formé en laminant une feuille de couche externe ;
agençant une pluralité de couches internes (18) à l'intérieur du conduit (16), chacune de la pluralité de couches internes s'étendant longitudinalement de la première extrémité à la deuxième extrémité ; et
intermaillant la longueur du fil en couches (100) pour construire la pièce de matériau lesté,
dans lequel la longueur de fil en couches, d'elle-même, est configurée et suffisamment lestée pour effectuer la thérapie par pression profonde sur une personne lorsque la couverture de thérapie par pression profonde est sur le corps d'une personne et dans lequel la pièce de matériau lesté est configurée pour peser entre 2,27 kg et 20,41 kg (5 livres et 45 livres) y compris.

14. Procédé selon la revendication 13, dans lequel :
le tube externe (1) a un diamètre s'étendant perpendiculairement à une direction longitudinale,
la pièce de matériau lesté a une longueur, une largeur et une hauteur ; et
la hauteur de la couverture de thérapie par pression profonde est égale au diamètre du tube externe.

15. Procédé selon la revendication 13, comprenant le fait de :
plier une feuille interne autour d'un axe de pliage qui s'étend longitudinalement de la première extrémité à la deuxième extrémité afin de créer au moins l'une de la pluralité de couches internes.
